# EUROPEAN PATENT APPLICATION

(11) **EP 4 556 556 A1**
(43) Date of publication of application: **21.05.2025**
(21) Application number: 23947831.6
(22) Date of filing: 03.11.2023
(51) Int. Cl.: C12N 5/077

(54) **METHOD FOR PURIFICATION AND PRODUCTION OF VENTRICULAR CARDIOMYOCYTES**

(30) Priority: 21.09.2023 KR 20230126632
(71) Applicant: T&R Biofab Co., Ltd., Siheung-si Gyeonggi-do 15111 (KR)
(72) Inventor: PARK, Soon-jung, Seoul 02638 (KR); MOON, Sung-hwan, Guri-si Gyeonggi-do 11940 (KR); PARK, Na Kong, Goyang-si Gyeonggi-do 10427 (KR)
(74) Representative: Plougmann Vingtoft a/s
(86) International application number: PCT/KR2023/017440
(87) International publication number: WO 2025/063371

(57) **Abstract**

Methods for purifying and producing ventricular cardiomyocytes are provided, wherein the methods utilize cell surface markers IL1RL1, CD81, and CD59 for marker-based separation of cardiomyocytes, enabling obtaining high-purity ventricular cardiomyocytes through physical separation methods.

## Description

### Technical field

The present invention relates to methods for purifying and producing ventricular cardiomyocytes. According to the present invention, a high concentration of ventricular cardiomyocytes can be obtained through a physical method using the cell surface markers CD81, IL1RL1, and CD59.

### Background art

Myocardial infarction refers to a condition in which cardiomyocytes undergo necrosis due to a reduced supply of oxygen and nutrients to the heart either partially or entirely, and cardiac failure refers to a condition resulting from an insufficient supply of blood due to impaired heart function. The myocardial infarction and cardiac failure may be caused due to the loss of cardiomyocytes (cardiac heart muscle cells).

The incidences of myocardial infarction and heart failure are increasing globally, but damaged or failing hearts cannot recover by themselves, leaving chemical therapies for these diseases to only delay disease progression.

Heart transplantation may be a fundamental therapy for restoring heart functions, but it may cause issues, such as a shortage of organ donors, medical ethics, and physical and financial burdens on patients.

For these reasons, cardiomyocytes are derived from stem cells, such as induced pluripotent stem cells (iPSCs) or embryonic stem cells (ESCs), and are used in regenerative medicine for cardiac diseases. Cardiomyocytes differentiated from stem cells may include three subtypes of cardiomyocytes: nodal, atrial, and ventricular cardiomyocytes, and may additionally include non-cardiomyocytes, such as undifferentiated cells.

In the separation of cardiomyocytes from these cells, cell surface markers specifically expressed in cardiomyocytes among the cell surface markers discovered so far have been searched, and representatively, CD177z, CD42, CD236a, SIRPA, CD71, and the like have been suggested. However, the surface markers discovered so far are only used as markers for purification for cardiomyocytes, and do not distinguish the types and functions of atrial and ventricular cells.

The ventricular cardiomyocytes may be directly associated with cardiac contraction in myocardial infarction. That is, it may be more important to separate pure ventricular cardiomyocytes in the cell regeneration therapy for heart function restoration. Moreover, arrhythmia may be caused when differentiated cardiomyocytes with different subtypes of cardiomyocytes are transplanted into disease models. Therefore, there is an urgent need to develop a technique to purify only pure ventricular cardiomyocytes.

### Disclosure of invention

### Technical Problem

In order to separate and purify ventricular cardiomyocytes in a highly concentrated state from a ventricular cardiomyocyte-containing cell group, the present inventors developed methods for purifying and producing ventricular cardiomyocytes by identifying that a single cell unit of ventricular cardiomyocytes can be effectively separated and purified when only CD81, IL1RL1, and CD59 positive cells are separated through a physical method.

An aspect of the present invention is to provide a method for purifying ventricular cardiomyocytes.

Another aspect of the present invention is to provide a method for producing ventricular cardiomyocytes.

### Solution to Problem

The present invention relates to methods for purifying and producing ventricular cardiomyocytes, and according to the present invention, a high concentration of ventricular cardiomyocytes can be obtained through a physical method using the cell surface markers CD81, IL1RL1, and CD59.

Hereinafter, the present invention will be described in more detail.

In accordance with an aspect of the present invention, there is provided a method for purifying ventricular cardiomyocytes, the method including: i) identifying the expression of at least one marker selected from the group consisting of IL1RL1, CD81, and CD59, in cardiomyocytes; and ii) separating cells positive for the expression, as identified in step i), of at least one marker selected from the group consisting of IL1RL1, CD81, and CD59, at the cellular level.

As used herein, the term "cardiomyocytes" refers to muscle cells constituting the heart wall. Cardiomyocytes may be classified into nodal cardiomyocytes, atrial cardiomyocytes, and ventricular cardiomyocytes according to the structure in the heart. The cardiomyocytes may be damaged or destroyed when exposed to stress, such as myocardial infarction or myocarditis. The damage or destruction of the cardiomyocytes results in a decline in myocardial function, which may cause heart diseases. Therefore, cardiomyocytes differentiated from pluripotent stem cells may be used in regenerative cell therapy for heart function restoration or heart disease treatment.

In cell regenerative therapy for myocardial infarction, it may be more important to separate pure ventricular cardiomyocytes among differentiated cardiomyocytes. The reason is that the purity of ventricular cardiomyocytes may affect the effect of cell regenerative therapy. For instance, in cell regenerative therapy, the use of different subtypes of cardiomyocytes together or the incorporation of immature cardiomyocytes or general myocytes may result in a poor prognosis compared with using pure ventricular cardiomyocytes. Hence, the purification of only ventricular cardiomyocytes among cardiomyocytes differentiated from stem cells is essential in regenerative cell therapy.

In the present invention, ventricular cardiomyocytes may be derived from stem cells, but are not limited thereto.

In the present invention, the stem cells may be human pluripotent stem cell (hPSCs), and for example, may be induced pluripotent stem cells (iPSCs) or embryonic stem cells (ESCs), but are not limited thereto.

As used herein, the term "marker" refers to a marker protein or a marker gene, which is a protein or a gene thereof that is specifically expressed on the cell surface, cytoplasm, and/or nucleus of a given cell, for example, the cell surface.

In the present invention, the markers in step i) and step ii) may further include CD151, and therefore, can further increase the purification efficiency of ventricular cardiomyocytes.

In the present invention, at least one marker selected from the group consisting of IL1RL1, CD81, CD59, and CD151 is used as a marker for purifying ventricular cardiomyocytes from a cardiomyocyte-containing cell population.

In the present invention, cells not expressing the marker, that is, cells negative for expression of the marker may be undifferentiated stem cells or embryoid bodies, or nodal cardiomyocytes or atrial cardiomyocytes.

In the present invention, the method for purifying ventricular cardiomyocytes may include: i) identifying the expression of at least one marker selected from the group consisting of IL1RL1, CD81, and CD59, and the marker CD151, in cardiomyocytes; and ii) separating cells positive for the expression, as identified in step i), of at least one marker selected from the group consisting of IL1RL1, CD81, and CD59, as well as the marker CD151, at the cellular level.

In the present invention, the method for purifying ventricular cardiomyocytes may further include, before step i), separating cells positive for expression of CD71 in cardiomyocytes, at the cellular level, to purify only contracting cells.

In the present invention, contracting cardiomyocytes can be analyzed for a change in the level of contracting ability according to the drug response, and thus it is preferable to separate and use only the contracting cardiomyocytes during the research using cardiomyocytes, such as cardiotoxicity drug evaluation of new drug candidates. This can also be applied as a cell therapy product for heart failure using contracting cardiomyocytes, and thus it may also be important to separate and distinguish contracting cardiomyocytes and non-contracting cardiomyocytes among differentiated cardiomyocytes.

In the present invention, CD71 may be used as a surface marker that is specifically expressed in contracting cardiomyocytes. Therefore, when cardiomyocytes are stained with the marker CD71, cells not expressing CD71, that is, CD71-negative cells may be non-contracting cardiomyocytes.

Cardiomyocytes that have not undergone the method for purifying ventricular cardiomyocytes according to the present invention may have a purity of ventricular cardiomyocytes of 10 to 70%, 10 to 60%, 10 to 50%, 20 to 70%, 20 to 60%, 20 to 50%, 30 to 70%, 30 to 60%, 30 to 50%, 40 to 70%, 40 to 60%, or 40 to 50%.

Cardiomyocytes that have undergone the method for purifying ventricular cardiomyocytes according to the present invention may have a purity of ventricular cardiomyocytes of 70 to 95%, 70 to 92%, 70 to 90%, 70 to 85%, 70 to 80%, 73 to 95%, 73 to 92%, 73 to 90%, 73 to 85%, 73 to 80%, 80 to 95%, 80 to 92%, 80 to 90%, or 80 to 85%.

In accordance with still another aspect of the present invention, there is provided a method for producing ventricular cardiomyocytes, the method including: i) inducing the differentiation of stem cells into cardiomyocytes; ii) identifying the expression of at least one marker selected from the group consisting of IL1RL1, CD81, and CD59, in the cardiomyocytes; and iii) separating cells positive for the expression, as identified in step ii), of at least one marker selected from the group consisting of IL1RL1, CD81, and CD59, at the cellular level.

In the present invention, the stem cells may be human pluripotent stem cells (hPSCs), for example, induced pluripotent stem cells (iPSCs) or embryonic stem cells (ESCs), but are not limited thereto.

In the present invention, the markers in step ii) and step iii) may further include CD151, so that ventricular cardiomyocytes with higher purity can be produced.

In the present invention, at least one marker selected from the group consisting of IL1RL1, CD81, CD59, and CD151 is used as a marker for purifying ventricular cardiomyocytes from a cardiomyocyte-containing cell population.

In the present invention, cells not expressing the marker, that is, cells negative for expression of the marker may be undifferentiated stem cells or embryoid bodies, or nodal cardiomyocytes or atrial cardiomyocytes.

In the present invention, the method for producing ventricular cardiomyocytes, the method including: i) inducing the differentiation of stem cells into cardiomyocytes; ii) identifying the expression of at least one marker selected from the group consisting of IL1RL1, CD81, and CD59, and the marker CD151 in the cardiomyocytes; and iii) separating cells positive for the expression, as identified in step ii), of at least one marker selected from the group consisting of IL1RL1, CD81, and CD59, and the marker CD151, at the cellular level.

In the present invention, the method for producing ventricular cardiomyocytes may include, between steps i) and ii), primarily purifying cardiomyocytes by using a metabolism control medium, and as a result, the purification efficiency of cardiomyocytes can be further improved due to the removal of cells other than cardiomyocytes.

In the present invention, the metabolism control medium may contain human serum albumin and lactate.

In the present invention, the metabolism control medium may contain human serum albumin and lactate while excluding glucose, and the purification efficiency of cardiomyocytes can be further improved by creating an environment where cells other than cardiomyocytes are inactive.

In the present invention, the method for producing ventricular cardiomyocytes may include, between steps i) and ii), maturing cardiomyocytes by using a cardiomyocyte maturation inducing medium, and as a result, the maturation period of cardiomyocytes can be shortened.

In the present invention, the cardiomyocyte maturation inducing medium may contain at least one selected from the group consisting of B27 supplement (B27), 3,3',5-triiodo-L-thyronine (T3), and dexamethasone (Dex).

In the present invention, the cardiomyocyte maturation inducing medium may contain at least one selected from the group consisting of B27 supplement (B27), 3,3',5-triiodo-L-thyronine (T3), and dexamethasone (Dex), which may be added to RPMI1640 medium.

In the present invention, the cardiomyocyte maturation inducing medium may contain B27 at a concentration of 0.1 to 5 wt%, 0.1 to 4 wt%, 0.1 to 3 wt%, 0.1 to 2 wt%, 0.5 to 5 wt%, 0.5 to 4 wt%, 0.5 to 3 wt%, 0.5 to 2 wt%, 1 to 5 wt%, 1 to 4 wt%, 1 to 3 wt%, or 1 to 2 wt%.

In the present invention, the cardiomyocyte maturation inducing medium may contain T3 at a concentration of 5 to 50 ng/ml, 5 to 40 ng/ml, 5 to 30 ng/ml, 5 to 20 ng/ml, 10 to 50 ng/ml, 10 to 40 ng/ml, 10 to 30 ng/ml, or 10 to 20 ng/ml.

In the present invention, the cardiomyocyte maturation inducing medium may contain Dex at a concentration of 1 to 10 nM, 1 to 9 nM, 1 to 8 nM, 1 to 7 nM, 1 to 6 nM, 1 to 5 nM, 3 to 10 nM, 3 to 9 nM, 3 to 8 nM, 3 to 7 nM, 3 to 6 nM, or 3 to 5 nM.

In the present invention, step ii) may be performed after the cardiomyocytes differentiation-induced in step i) are cultured for 14 to 28 days, 14 to 26 days, 14 to 24 days, 14 to 22 days, 14 to 20 days, 14 to 18 days, or 14 to 16 days, for example, 14 days, so that high-purity ventricular cardiomyocytes can be obtained.

In the present invention, step ii) may be performed after the cardiomyocytes differentiation-induced in step i) are primarily purified using a metabolism control medium and then cultured with a cardiomyocyte maturation inducing medium for 14 to 28 days, 14 to 26 days, 14 to 24 days, 14 to 22 days, 14 to 20 days, 14 to 18 days, or 14 to 16 days, for example, 14 days, so that high-purity ventricular cardiomyocytes can be obtained.

In the present invention, the method for producing ventricular cardiomyocytes may further include, between steps i) and ii), separating cells positive for expression of CD71 in cardiomyocytes, at the cellular level, to purify only contracting cardiomyocytes.

In the present invention, contracting cardiomyocytes can be analyzed for a change in the level of contracting ability according to the drug response, and thus it is preferable to separate and use only the contracting cardiomyocytes during the research using cardiomyocytes, such as cardiotoxicity drug evaluation of new drug candidates. This can also be applied as a cell therapy product for heart failure using contracting cardiomyocytes, and thus it may also be important to separate and distinguish contracting cardiomyocytes and non-contracting cardiomyocytes among differentiated cardiomyocytes.

In the present invention, CD71 may be used as a surface marker that is specifically expressed in contracting cardiomyocytes. Therefore, when cardiomyocytes are stained with the marker CD71, cells not expressing CD71, that is, CD71-negative cells may be non-contracting cardiomyocytes.

High-purity ventricular cardiomyocytes can be obtained by the method for producing ventricular cardiomyocytes according to the present invention, and specifically, the purity of the ventricular cardiomyocytes may be 70 to 95%, 70 to 92%, 70 to 90%, 70 to 85%, 70 to 80%, 73 to 95%, 73 to 92%, 73 to 90%, 73 to 85%, 73 to 80%, 80 to 95%, 80 to 92%, 80 to 90%, or 80 to 85%.

### Advantageous Effects of Invention

The present invention relates to methods for purifying and producing ventricular cardiomyocytes, and according to the present invention, a high concentration of ventricular cardiomyocytes can be obtained through a physical method using the cell surface markers CD81, IL1RL1, and CD59.

### Brief description of drawings

FIG. 1 illustrates a schematic diagram showing cardiomyocyte differentiation and ventricular cardiomyocyte purification according to an embodiment of the present invention.
FIG. 2 illustrates a graph showing the results of analyzing the action potential of cardiomyocytes before secondary purification using ventricular cardiomyocyte purification markers according to an embodiment of the present invention.
FIG. 3 illustrates a graph showing the results of RNA-seq analysis performed to secure a list of ventricular cardiomyocyte purification markers according to an embodiment of the present invention.
FIG. 4 shows cell images after secondary purification using ventricular cardiomyocyte purification markers, and an action potential measurement method according to an embodiment of the present invention.
FIG. 5 illustrates graphs showing the results of analyzing the action potential of cardiomyocytes after secondary purification using ventricular cardiomyocyte purification markers according to an embodiment of the present invention.
FIG. 6 illustrates graphs identifying gene expression patterns before and after ventricular cardiomyocyte purification according to an embodiment of the present invention.
FIG. 7 shows the results of confirming that only contracting cardiomyocytes in differentiated cardiomyocytes can be specifically labeled through CD71 staining (FIG. 7A) and the results of flow cytometry analysis of SIRPA marker cells as control and CD71 marker cells (FIG. 7B).

### Best mode for carrying out the invention

The present invention relates to a method for purifying ventricular cardiomyocytes, the method including: i) identifying the expression of at least one marker selected from the group consisting of IL1RL1, CD81, and CD59, in cardiomyocytes; and ii) separating cells positive for the expression, as identified in step i), of at least one marker selected from the group consisting of IL1RL1, CD81, and CD59, at the cellular level.

### Mode for Carrying out the Invention

Hereinafter, the present disclosure will be described in more detail by the following exemplary embodiments. However, these exemplary embodiments are used only for illustration, and the scope of the present disclosure is not limited by these exemplary embodiments.

### Example 1: Selection and Efficacy of Ventricular Cardiomyocyte Purification Markers

### 1-1. Production of human induced pluripotent stem cells (hiPSCs)

A human induced pluripotent stem cell (hiPSC) line was created from somatic cells of healthy individuals with completed donor consent forms by using a vector- and virus-free reprogramming kit (Stemgent, StemRNA^{™} 3rd Gen Reprogramming Kit) according to the manufacturer's protocol.

### 1.2 Induction of differentiation of human induced pluripotent stem cells (hiPSCs) into cardiomyocytes

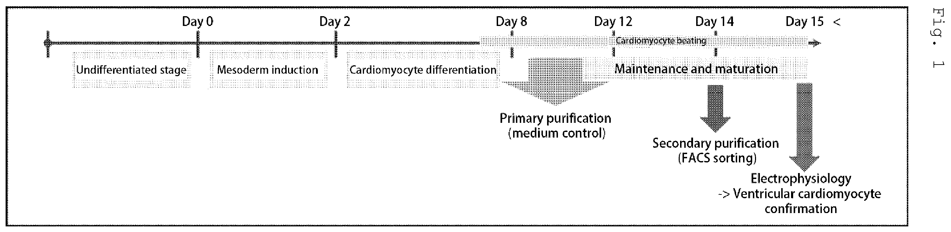

The differentiation of cardiomyocytes was induced from the human induced pluripotent stems by the following method according to the schedule in FIG. 1. The human induced pluripotent stem cell (hiPSC) line was maintained in an undifferentiated state on Matrigel using iPS-BREW XF medium (StemMACS TM, Mitenyi Biotec) for 4 days. For initial differentiation, the cultured stem cells were sequentially treated with the small molecule compounds CHIR 99021 and Wnt-C59, and the cells were differentiated and cultured for 4 days in cardiomyocyte differentiation medium (CDM; RPMI1640 (ThermoFisher Scientific) + 500 µg/ml human serum albumin (Sigma Aldrich) + 213 µg/ml ascorbic acid (Sigma Aldrich)) to induce differentiation into contracting cardiomyocytes.

After 8 days of differentiation induction, contracting cardiomyocytes were observed, and the cells were primarily purified for 3 days using a metabolic control medium containing human serum albumin (Sigma, 2%) and lactate (Sigma, 1 mM/ml) in glucose-free RPMI1640 medium.

Then, the medium was replaced with a cardiomyocyte maturation inducing medium (RPMI1640 + B27 (B27 supplement, Gibco, 1%) + T3 (3,3',5-triiodo-L-thyronine, Sigma, 10 ng/ml) + dexamethasone (Stemcell technology, 5 nM) for 14 days.

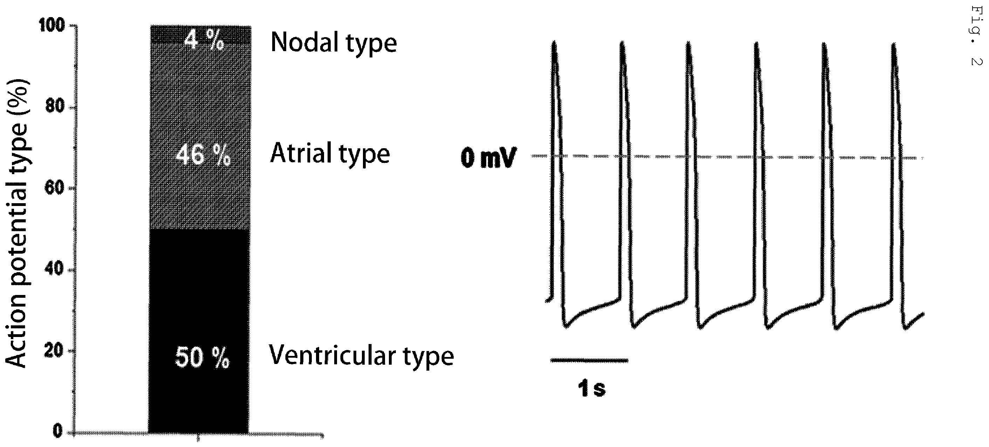

The cultured cardiomyocytes were separated into single cells by using TyprLE-select (Gibco), and then the voltages and currents of all three types of cardiomyocytes, including ventricular, atrial, and nodal cardiomyocytes, were recorded through an electrophysiological analysis method (patch clamp). The results are shown in FIG. 2. As shown in FIG. 2, the action potential of cardiomyocytes after only the primary purification showed a distribution of 4% nodal cardiomyocytes, 46% atrial cardiomyocytes, and 50% ventricular cardiomyocytes, mixed together.

### 1-3. Selection of ventricular cardiomyocyte purification marker through RNA single gene analysis (single cell array)

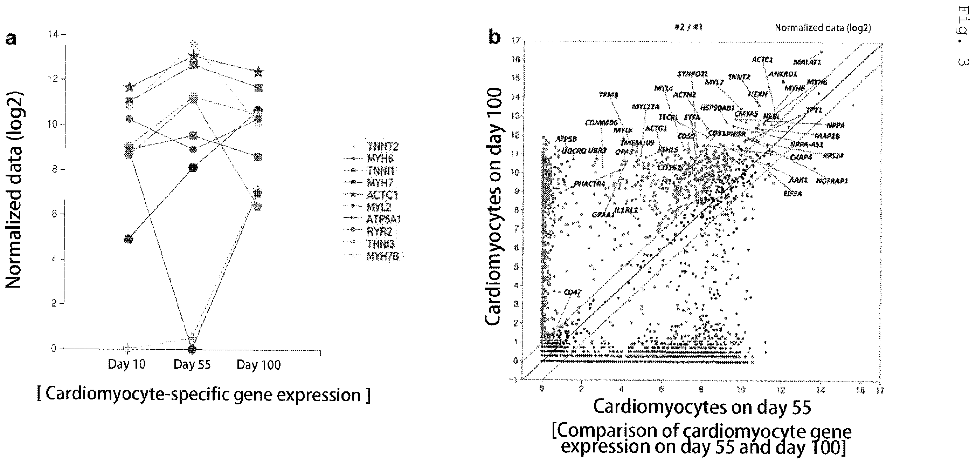

The differentiation-induced cardiomyocytes were primarily purified, and cultured in a maturation inducing medium for 10, 55, and 100 days, separately, and manually separated into 100 single cell units, separately. Then, each cell was lyzed to obtain a cell extract, followed by global transcriptome assay. From the results of the global transcriptome analysis, the hierarchical clustering analysis results of the global gene expression profile were derived using average linkage, and the results are shown in FIG. 3A.

As a result, as can be seen in FIG. 3A, all the analyzed cells were cardiomyocytes through changes in cardiomyocyte-specific genes in the cardiomyocytes cultured for 10, 55, and 100 days.

Cardiomyocyte genes and ventricular cardiomyocyte genes were primarily separated from the analyzed genes, and then, among the genes showing a difference in expression level between 55 and 100 days of culture, the genes encoding cell surface markers (CDs) of the increased genes were analyzed, and the results are shown in FIG. 3B. Through the results in FIG. 3, CD59, IL1RL1, CD81, and CD151 as surface markers specific in ventricular cardiomyocytes were selected.

### 1-4. Efficacy of ventricular cardiomyocyte purification markers

First, among the genes that showed a difference in expression level in the differentiation-induced cardiomyocytes, genes encoding cell surface markers (CDs) were compared and analyzed from the results analyzed in the above examples. For efficient purification of ventricular cardiomyocytes, pure cardiomyocytes were primarily purified using a metabolism control medium.

It was investigated whether CD59, IL1RL1, CD81, and CD151 could be used as markers to specifically select only ventricular cardiomyocytes among cardiomyocytes differentiated from hPSCs. Hence, the cardiomyocytes were cultured in a maturation inducing medium for 14 days, stained with CD59, IL1RL1, CD81, and CD151, and then secondarily purified using a flow cytometer.

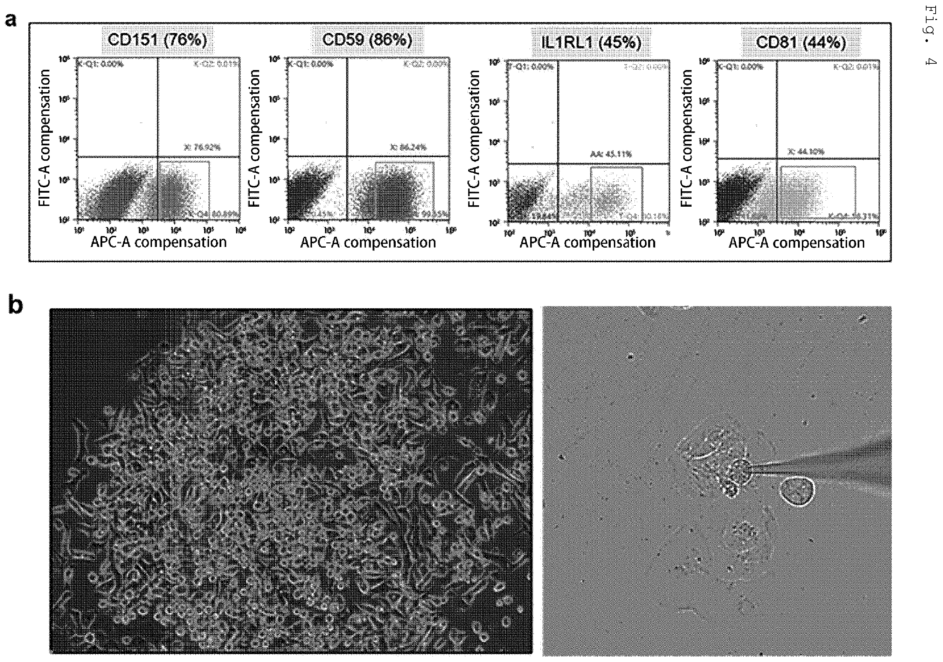

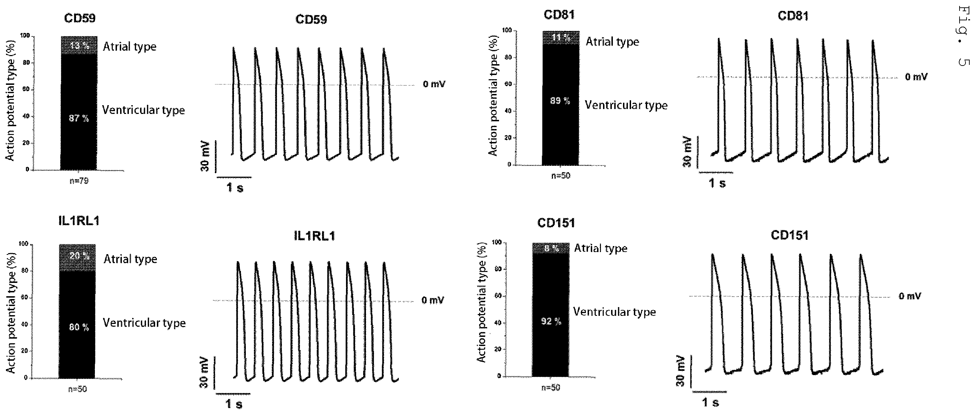

The cardiomyocytes separated and purified by the markers CD59, IL1RL1, CD81, and CD151 were cultured in a maturation inducing medium for 24 hours, and then as shown in FIG. 4A, the contracting of cells purified with the markers were confirmed. Thereafter, as shown in FIG. 4B, the activation potentials were recorded using patch clamping, and the results are shown in FIG. 5.

As a result, as shown in FIG. 5, the active potential of cardiomyocytes separated and purified by the marker CD59 showed a distribution of 87% of ventricular cardiomyocytes, the active potential of cardiomyocytes separated and purified by the marker IL1R1 showed a distribution of 80% of ventricular cardiomyocytes, the active potential of cardiomyocytes separated and purified by the marker CD81 showed a distribution of 89% of ventricular cardiomyocytes, and the active potential of cardiomyocytes separated and purified by the marker CD151 showed a distribution of 92% of ventricular cardiomyocytes.

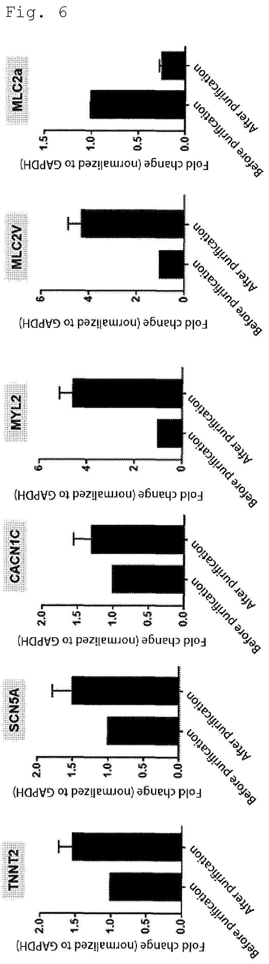

In addition, the cardiomyocytes after only primary purification and the cardiomyocytes after secondary purification using the CD59 marker were further subjected to gene analysis, and the results are shown in FIG. 6. As shown in FIG. 6, the cardiomyocytes after secondary purification using the CD59 marker (after sorting), compared with the cardiomyocytes after only primary purification (before sorting), showed increases in TNNT2, a cardiomyocyte-specific gene, and SCN5A and CACN1C, ion channel genes involved in cardiomyocytes, increases in MYL2 and MLC2V, cardiomyocyte-specific genes, and a decrease in MLC2a, an atrial cardiomyocytes.

It was therefore identified that ventricular cardiomyocytes can be specifically purified and separated in cardiomyocytes through CD59, IL1RL1, CD81, and CD151, which were markers selected in the present invention.

### Example 2: Efficacy of contracting cardiomyocyte purification marker CD71

The efficacy of the marker CD71 for specifically selecting only contracting cells among cardiomyocytes differentiated from hPSCs was investigated (Patent Document KR 10-1916902 B1). The cardiomyocytes differentiated from hPSCs were stained by treatment with anti-CD71 antibody, visualized with anti-CD71 antibody, and then observed under a microscope. In addition, in order to investigate whether CD71 could be used as a significant surface marker, the cardiomyocytes were labeled with SIRPA or CD71, known to be usable as cardiomyocyte identification markers, to analyze the fluorescence intensity by using a flow cytometer (FACS).

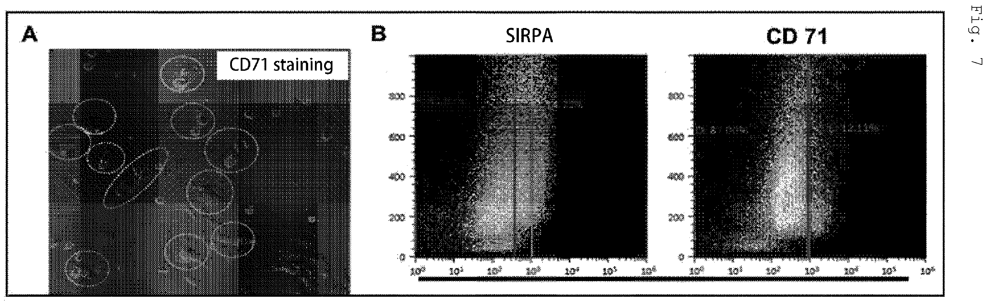

As a result, as shown in FIG. 7, it was confirmed that CD71 can be used as a surface marker specific for contracting cardiomyocytes (FIG. 7A), and the flow cytometry analysis results confirmed that the CD71 surface marker showed a similar pattern to SIRPA, a cardiomyocyte surface factor (FIG. 7B).

Since SIRPA is known to be a factor specific for cardiomyocytes regardless of contracting or non-contracting cells, only contracting cardiomyocytes can be specifically selected using the marker CD71.

### Industrial Applicability

The present invention relates to methods for purifying and producing ventricular cardiomyocytes, and according to the present invention, a high concentration of ventricular cardiomyocytes can be obtained through a physical method using the cell surface markers CD81, IL1RL1, and CD59.

## Claims

1. A method for purifying ventricular cardiomyocytes, the method comprising:
i) identifying the expression of at least one marker selected from the group consisting of IL1RL1, CD81, and CD59, in cardiomyocytes; and
ii) separating cells positive for the expression, as identified in step i), of at least one marker selected from the group consisting of IL1RL1, CD81, and CD59, at the cellular level.

2. The method of claim 1, wherein the cardiomyocytes are derived from stem cells.

3. The method of claim 2, wherein the stem cells are human pluripotent stem cells (hPSCs).

4. The method of claim 1, wherein the markers in step i) and step ii) further include CD151.

5. The method of claim 1, further comprising, before step i), separating cells positive for expression of CD71 in cardiomyocytes, at the cellular level, to purify only contracting cardiomyocytes.

6. A method for producing ventricular cardiomyocytes, the method comprising:
i) inducing the differentiation of stem cells into cardiomyocytes;
ii) identifying the expression of at least one marker selected from the group consisting of IL1RL1, CD81, and CD59, in the cardiomyocytes; and
iii) separating cells positive for the expression, as identified in step ii), of at least one marker selected from the group consisting of IL1RL1, CD81, and CD59, at the cellular level.

7. The method of claim 6, wherein the stem cells are human pluripotent stem cells (hPSCs).

8. The method of claim 6, wherein the markers in step ii) and step iii) further include CD151.

9. The method of claim 6, further comprising, between steps i) and ii), primarily purifying cardiomyocytes by using a metabolism control medium.

10. The method of claim 9, wherein the metabolism control medium contains human serum albumin and lactate.

11. The method of claim 6, further comprising, between steps i) and ii), maturing cardiomyocytes by using a cardiomyocyte maturation inducing medium.

12. The method of claim 11, wherein the cardiomyocyte maturation inducing medium contains at least one selected from the group consisting of B27 supplement (B27), 3,3',5-triiodo-L-thyronine (T3), and dexamethasone (Dex) .

13. The method of claim 6, wherein step ii) is performed after culturing the cardiomyocytes differentiated in step i) for 14 days to 28 days.

14. The method of claim 6, further comprising, between steps i) and ii), separating cells positive for expression of CD71 in cardiomyocytes, at the cellular level, to purify only contracting cardiomyocytes.
